# EUROPÄISCHE PATENTSCHRIFT

(11) **EP 0 360 222 B1**
(45) Veröffentlichungstag und Bekanntmachung des Hinweises auf die Patenterteilung: **10.03.1993**
(21) Anmeldenummer: 89117307.2
(22) Anmeldetag: 19.09.1989
(51) Int. Cl.: C07C 229/12, C07C 227/00

(54) **Verfahren zur Herstellung von gamma-Butyrobetain**
Process for preparing gamma-butyrobetaine
Procédé pour la préparation de gamma-butyrobétaine

(30) Priorität: 21.09.1988 CH 3514/88
(43) Veröffentlichungstag der Anmeldung: 28.03.1990
(73) Patentinhaber: LONZA AG, CH-4002 Basel (CH)
(72) Erfinder: Hardt, Peter, Dr., Visp (Kanton Wallis) (CH); Stravs, Andrej, Dr., Ried bei Brig (Kanton Wallis) (CH); Abgottspon, Pius, Stalden (Kanton Wallis) (CH)
(74) Vertreter: Weinhold, Peter, Dr.

(56) Entgegenhaltungen:
- EP-A- 0 244 370
- DE-A- 1 903 076
- DE-A- 2 305 636
- JOURNAL OF THE CHEMICAL SOCIETY, Teil I, 1959, London G.AKSNES et al. "Kinetic salt effects and mechanism in the hydrolysis of positively charged esters" Seiten 103-107

## Beschreibung

γ-Butyrobetain findet zunehmende Verwendung als Ausgangsprodukt für die mikrobiologische Herstellung von L-Carnitin. Die Erfindung beschreibt ein neues Verfahren zu dessen Herstellung.

Laborverfahren zur Synthese der einzelnen Zwischenstufen sind ausreichend beschrieben.
Gemäss DE-OS 27 51 134 lässt sich γ-Butyrolacton mit Thionylchlorid und Methanol in 91% Ausbeute in den γ-Chlorbuttersäuremethylester umsetzen. Nachteilig bei diesen Verfahren ist die notwendige Entsorgung des anfallenden SO₂.
Aus der DE-OS 19 03 076 geht hervor, dass γ-Butyrolacton sich mit trockener Salzsäure und Methanol bei 4 Stunden Rückfluss und nach einwöchigem Stehenlassen der Reaktionslösung in den γ-Chlorbuttersäuremethylester überführen lässt.
Der DE-OS 19 39 759 ist zu entnehmen, dass γ-Butyrolacton in einem Zweistufenverfahren (erste Stufe mit Zinkchlorid, Salzsäure, zweite Stufe mit Methanol unter Rückflussbedingungen) mit einer Ausbeute von 90 bis 95% in den γ-Chlorbuttersäuremethylester überführt werden kann. Von grossem Nachteil ist aber der Anfall von nicht rezirkulierbarem und das Abwasser schwer belastenden Zinksalz.
Von Aksnes et al. J.Chem.Soc.1959, S.103f. ist weiter bekannt, dass γ-Brombuttersäuremethylester durch Erhitzen mit alkoholischem Trimethylamin in einer Ausbeute von lediglich 20% in den 4-Trimethylammoniumbuttersäuremethylester umgesetzt werden kann.

Die vorbenannten Verfahrensstufen ergeben also entweder höchst unbefriedigende Ausbeuten oder sind aufgrund der Entsorgungsproblematik von anfallenden Nebenprodukten nicht im industriellen Massstab durchführbar.
Es bestand daher die Aufgabe, ein einfaches, im grosstechnischen Massstab durchführbares Verfahren zur Herstellung von γ-Butyrobetain aus Butyrolacton zu entwickeln, das sowohl gute Ausbeuten liefert und vom ökologischen Aspekt her unbedenklich ist.

Die Aufgabe konnte gelöst werden mit dem erfindungsgemässen Verfahren nach Patentanspruch 1. Dabei wird in einem ersten Schritt γ-Butyrolacton mit Chlorwasserstoff in die γ-Chlorbuttersäure umgesetzt. Zweckmässig arbeitet man bei einem Druck von 1 bis 25 bar, vorzugsweise bei 8 bis 20 bar. Die Reaktionstemperatur kann dabei zwischen 40 und 150°C, vorzugsweise zwischen 80 und 120°C variieren.
Der Chlorwasserstoff wird üblicherweise in einem Ueberschuss von 5 bis 40%, bezogen auf das γ-Butyrolacton eingesetzt. Die Umsetzung in die γ-Chlorbuttersäure dauert ca. 2 bis 10 Stunden und verläuft in der Regel praktisch quantitativ.
Es ist ein Vorteil des erfindungsgemässen Verfahrens, dass die γ-Chlorbuttersäure nicht isoliert werden muss, sondern der Reaktionslösung der ersten Stufe direkt der für die Esterbildung notwendige Alkohol zugegeben werden kann. Der Chlorwasserstoffüberschuss aus der ersten Stufe kann dabei direkt als Veresterungskatalysator fungieren. Es kann sich aber als notwendig erweisen, zusätzlichen Chlorwasserstoff für die Veresterung bereitzustellen.

Zur Veresterung werden zweckmässig Methanol, Ethanol, Propanole oder Butanole, vorzugsweise Methanol oder Ethanol angewendet.

Die Veresterungsreaktion verläuft vorteilhaft bei einem Druck von 1 bis 15 bar, besonders vorteilhaft bei 1 bis 10 bar und einer Temperatur von zweckmässig 40 bis 150°C, vorzugsweise 70 bis 120°C.
Es ist aber auch möglich, die Umsetzung des γ-Butyrolactons in den γ-Chlorbuttersäureester in einer Stufe durchzuführen. Dazu werden die Reaktanden γ-Butyrolacton, Chlorwasserstoff und der entsprechende Alkohol zusammengegeben und bei einem Druck von 1 bis 10 bar und Temperaturen von 40 bis 150°C direkt zum entsprechenden γ-Chlorbuttersäureester umgesetzt.
In der dritten Stufe erfolgt die Ueberführung des γ-Chlorbuttersäureesters mit Trimethylamin in das Trimethylammoniumbuttersäureesterchlorid. Zweckmässig wird diese Reaktion bei einem Druck von 1 bis 10 bar und einer Temperatur von 20 bis 180°C, vorteilhaft bei 80 bis 150°C durchgeführt.
Das Trimethylamin kann in einem leichten Ueberschuss oder in stöchiometrischen Mengen, vorzugsweise aber in einem leichten Ueberschuss, zugesetzt werden.
Es ist von Vorteil, als Lösungsmittel den dem Esterrest entsprechende Alkohol zu verwenden. Es ist aber durchaus möglich, Trimethylamin-lösliche Lösungsmittel, wie z.B. Toluol, einzusetzen.

Die Umsetzung in das γ-Trimethylammoniumbuttersäureesterchlorid dauert in der Regel 2 bis 6 Stunden und verläuft praktisch quantitativ.
Die Reaktionslösung kann wiederum direkt der letzten Stufe der Verseifung zugeführt werden.
Als Basen können im Prinzip alle für Verseifungen bekannten starken Basen herangezogen werden. Vorzugsweise gelangen aber wässrige Lösungen von anorganischen Basen, wie den Erdalkalioder Alkalihydroxiden (z.B. NaOH oder KOH) oder den Erdalkalioder Alkalicarbonaten (z.B. Natriumcarbonat), zur Anwendung. Eine Reaktionstemperatur von 20 bis 100°C hat sich für die Verseifung als vorteilhaft erwiesen.
Die Aufarbeitung bzw. Reinigung des γ-Butyrobetains aus der Reaktionslösung kann - dem weiteren Verwendungszweck angepasst - z.B. durch Entsalzen mittels Ionenaustauscher, spez. Kristallisationsmethoden oder durch Elektrodialyse erfolgen. Die letztere Methode wird erfolgreich eingesetzt, um eine vollständig entsalzte γ-Butyrobetainlösung zu erhalten, die gegebenenfalls nach Verdünnung direkt einer mikrobiologischen Carnitinsynthese zugeführt werden kann.
Das nach dem erfindungsgemässen Verfahren hergestellte γ-Butyrobetain weist eine Reinheit in der Regel von grösser 99,5% auf.
Im weiteren kann mit dem neuen Verfahren eine overall-Ausbeute γ-Butyrobetain bezogen auf γ-Butyrolacton von grösser 65% erzielt werden.

### Beispiel

### a) Herstellung von γ-Chlorbuttersäureethylester

51,7 kg (0,6 kMol) γ-Butyrolacton (100%ig) wurden in einem Emaildruckrührwerk vorgelegt. Das geschlossene System wurde bei gutem Rühren auf 100°C aufgeheizt, wobei ab 60°C insgesamt ca. 26,5 kg (0,72 kMol) HCl aufgepresst wurden. Bedingt durch die Exothermie stiegen die Temperatur und der Druck rasch an. Die Dosierung von HCl und die Heizleistung wurden derart geregelt, dass die Reaktion isotherm bei 100°C und isobar bei 11 bar Druck gefahren werden konnte. Es wurde solange dosiert bis kein HCl mehr aufgenommen wurde (ca. 5 bis 6 Stunden). Dann wurde auf 20°C abgekühlt und das restliche HCl abgelassen.
Der Reaktionslösung wurden darauf 62,3 kg (1,35 kMol) Ethanol zugegeben. Dann wurden nochmals 2 kg (0,055 kMol) HCl aufgepresst. Es wurde auf 100°C aufgeheizt und während 2 Stunden bei dieser Temperatur gehalten (Druck 6 bar) und dann auf 20°C abgekühlt. Die Lösung wurde darauf mit 92 kg Toluol versetzt und mit 23 kg wässriger NaOH (30%) basisch gestellt (pH 8 bis 8,5). Die Phasen wurden abgetrennt und die organische Phase mit 26 kg Wasser gewaschen. Die vereinigten Wasserphasen wurden nochmals mit 46 kg Toluol extrahiert. Die organische Phase wurde destilliert. Dabei wurde Toluol, Ethanol und Wasser abgetrennt. Als Blasenrückstand verblieben 87 kg Roh-γ-Chlorbuttersäureethylester (Gehalt 88%), der direkt in die Folgestufe eingesetzt werden konnte.
Ausbeute 85% bez. auf γ-Butyrolacton.

### b) Herstellung von γ-Butyrobetain

159 kg (0,92 kMol) Roh-γ-Chlorbuttersäureethylester Gehalt 88% und 107 kg (2,3 kMol) Ethanol wurden in einem Druckrührwerk vorgelegt. Dann wurden während 15 bis 30 Minuten 57 kg (0,96 kMol) Trimethylamin zudosiert. Die Temperatur stieg dabei auf 30 bis 50°C. Dann wurde auf 130°C aufgeheizt. Der Druck stieg dabei auf 5 bis 7 bar an und fiel dann wieder auf ca. 4 bar zurück. Nach Erreichen eines stabilen Drucks wurde auf 20°C abgekühlt und das restliche Trimethylamin entfernt.
Die Reaktionslösung wurde mit 138 kg wässriger NaOH (30%) auf pH grösser 11 gestellt. Während 1 Stunde wurde dann bei 60°C gehalten und der pH evtl. nachgestellt. Dann wurde auf 20°C abgekühlt und das ausgefallene NaCl abfiltriert. Nach destillativer Entfernung des überschüssigen Trimethylamins und Lösungsmittels wurde der Rückstand mit Wasser verdünnt. Diese Lösung wurde auf pH 8 gestellt, filtriert und mittels Elektrodialyse entsalzt. Die resultierende Lösung enthielt 32% γ-Butyrobetain entsprechend einer Ausbeute von 80%, bezogen auf γ-Chlorbuttersäureethylester. Gehalt 99,5% (HPLC-Bestimmung einer entwässerten Probe).

## Patentansprüche

1. Verfahren zur Herstellung von γ-Butyrobetain, dadurch gekennzeichnet, dass man γ-Butyrolacton mit Chlorwasserstoff in die γ-Chlorbuttersäure umsetzt, diese ohne zu isolieren mit Methanol, Ethanol, einem Propanol oder einem Butanol in den entsprechenden, γ-Chlorbuttersäureniederalkylester überführt mit Trimethylamin in das Trimethylammoniumbuttersäureniederalkylesterchlorid umsetzt und dieses ohne zu isolieren schliesslich mit einer Base zum Endprodukt verseift.

2. Verfahren nach Patentanspruch 1, dadurch gekennzeichnet, dass die Umsetzung in die γ-Chlorbuttersäure bei einem Druck von 1 bis 25 bar und einer Reaktionstemperatur von 40 bis 150°C erfolgt.

3. Verfahren nach Patentansprüchen 1 und 2, dadurch gekennzeichnet, dass die Umsetzung in den γ-Chlorbuttersäureester bei einem Druck von 1 bis 15 bar und einer Reaktionstemperatur von 40 bis 150°C erfolgt.

4. Verfahren nach Patentansprüchen 1 bis 3, dadurch gekennzeichnet, dass Methanol oder Ethanol eingesetzt wird.

5. Verfahren nach Patentansprüchen 1 bis 4, dadurch gekennzeichnet, dass γ-Butyrolacton mit Chlorwasserstoff und dem entsprechenden Alkohol in einer Stufe in den γ-Chlorbuttersäureniederalkylester überführt wird.

6. Verfahren nach Patentansprüchen 1-5, dadurch gekennzeichnet, dass die Umsetzung mit Trimethylamin bei einem Druck von 1 bis 10 bar und einer Temperatur von 20 bis 180°C durchgeführt wird.

## Claims

1. Process for preparing gamma-butyrobetaine, characterized in that gamma-butyrolactone is reacted with hydrogen chloride to gamma-chlorobutyric acid, the latter without isolation is reacted with methanol, ethanol, a propanol or a butanol to the corresponding lower alkyl gamma-chlorobutyrate, which latter is reacted with trimethyl amine to trimethyl ammonium butyric acid lower alkyl ester chloride, and this without isolation is finally saponified with a base to produce the end product.

2. Process according to patent claim 1, characterized in that the reaction to the gamma-chlorobutyric acid is carried out at a pressure of from 1 to 25 bar and at a reaction temperature of from 40 to 150°C.

3. Process according to patent claims 1 and 2, characterized in that the reaction into the gamma-chlorobutyrate is carried out at a pressure of from 1 to 15 bar and at a reaction temperature of from 40 to 150°C.

4. Process according to patent claims 1 to 3, characterized in that methanol or ethanol is used.

5. Process according to patent claims 1 to 4, characterized in that gamma-butyrolactone is reacted in one step with hydrogen chloride and the corresponding alcohol to the lower alkyl gamma-chlorobutyrate.

6. Process according to patent claims 1 to 5, characterized in that the reaction with trimethyl amine is carried out at a pressure of from 1 to 10 bar and at a temperature of from 20 to 180°C.

## Revendications

1. Procédé pour la préparation de γ-butyrobétaïne, caractérisé en ce que l'on fait réagir la γ-butyrolactone avec du gaz chlorhydrique dans l'acide γ-chlorobutyrique, que l'on transforme sans isolation avec le méthanol, l'éthanol, un propanol ou un butanol en l'alkylester inférieur de l'acide γ-chlorobutyrique correspondant et que l'on fait réagir avec de la triméthylamine dans le chlorure d'alkylester inférieur de l'acide triméthylammonium butyrique et pour finir que l'on saponifie celui-ci sans isolation avec une base pour donner le produit final.

2. Procédé selon la revendication 1, caractérisé en ce que la réaction dans l'acide γ-chlorobutyrique s'effectue à une pression de 1 à 25 bars et à une température réactionnelle de 40 à 150°C.

3. Procédé selon les revendications 1 et 2, caractérisé en ce que la réaction dans l'ester de l'acide γ-chlorobutyrique s'effectue à une pression de 1 à 15 bars et à une température réactionnelle de 40 à 150°C.

4. Procédé selon les revendications 1 à 3, caractérisé en ce que l'on met en oeuvre du méthanol ou de l'éthanol.

5. Procédé selon les revendications 1 à 4, caractérisé en ce que la γ-butyrolactone avec le gaz chlorhydrique et l'alcool correspondant est transformée en une étape en l'alkylester inférieur de l'acide γ-chlorobutyrique.

6. Procédé selon les revendications 1 à 5, caractérisé en ce que la réaction avec la triméthylamine s'effectue à une pression de 1 à 10 bars et une température de 20 à 180°C.
